# EUROPEAN PATENT APPLICATION

(11) **EP 4 095 502 A1**
(43) Date of publication of application: **30.11.2022**
(21) Application number: 21175698.6
(22) Date of filing: 25.05.2021
(51) Int. Cl.: G01L 19/14, A61B 5/0215

(54) **DEVICE FOR MEASURING PRESSURE OF A FLUID, TUBULAR STRUCTURE, METHOD FOR MANUFACTURING A DEVICE FOR MEASURING PRESSURE OF A FLUID AND METHOD FOR MEASURING PRESSURE OF A FLUID**

(71) Applicant: ETH Zurich, 8092 Zurich (CH)
(72) Inventor: HIEROLD, Christofer, 5400 Baden (CH); SCHMID DANERS, Marianne, Schaffhausen 8200 (CH); VON PETERSDORFF-KAMPEN, Kai, 8002 Zürich (CH); DUPUCH, Matthias, 8442 Hettlingen (CH); ENKE, Jonas, 8307 Effretikon (CH)

(57) **Abstract**

The invention relates to a device for measuring pressure of a fluid, and to a tubular structure, to a method for manufacturing a device for measuring pressure of a fluid, as well as to a method for measuring pressure of a fluid, in particular of blood.

The device (1) for measuring pressure of a fluid (3) comprises at least one sensor device (12) in a support member (10) comprising a cavity (11) filled or fillable with a pressure transmission fluid (30), in particular a working liquid, and inside the cavity (11) a sensor element (21), wherein the cavity (11) is closed on one side by a membrane (13), wherein the membrane (13) abuts against an abutment surface (15) of the support member (10) and at least one rim (132) of the membrane (13) is fixed to the abutment surface (15), and an inner area (Ai) restricted on its circumference (14) by the zone of fixation (152) of the membrane (13) to the abutment surface (15) is not fixed to the abutment surface (15), so that the inner area (Ai) of the membrane (13) is movable with regard to the abutment surface (15) and can elevate the abutment surface (15).

## Description

The invention relates to a device for measuring pressure of a fluid in a tubular structure, in particular of blood in an inflow cannula of a mechanical circulatory support device like a blood circulation assist device, comprising the device for measuring pressure of a fluid. Further, the invention relates to a tubular structure comprising the device for measuring pressure of a fluid, in particular a mechanical circulatory support like a Ventricular Assist Device or an Inflow device.

And, the invention relates to a method for manufacturing a device for measuring pressure of a fluid, in particular blood, as well as to a method for measuring pressure of a fluid, in particular of blood.

### BACKGROUND

Blood pressure and blood flow rates are important parameters for the control of mechanical circulatory support devices. Combined accurate total pressure in the ventricle and flow determination with one type of sensor in tubular structures, e.g., the inflow cannula of a ventricular assist device, are desirable in order to achieve space-saving solutions and to reduce complexity of manufacturing processes.
From literature, pressure sensor encapsulations integrated into ventricular assist devices are known for monitoring blood pressure for controlling the device output according to the patient's needs and/ or patient monitoring. However, said pressure sensor encapsulations are subject to temperature cross-sensitivity and drift phenomena, which originate from the viscoelastic/plastic properties of the polymer interface membrane.

At conventional positioning of the sensing interface inside a tubular structure, e.g., of a ventricular assist device, only measuring static pressure instead of the total pressure is carried out, so that due to the Bernoulli effect the measurement is influenced by flow rate. This can lead to deviation of the measured pressure to the actual ventricular pressure value relevant for the control system and/ or for patient monitoring.
While continuous measurement of pump flow enables patient monitoring and future automatic adaption of the pump output to the patients' perfusion need, current installations either rely on additional dedicated measurement systems or derivation of values of the flow rate which are based on evaluation of the pump motor's electricity consumption and rotational speed.

The pressure sensor encapsulation usually comprises a pressure deflectable interface membrane made from biocompatible polymer, separating the bloodstream essentially seamlessly from a cavity in which a sensor is embedded in a pressure transmission fluid (PTF). This fluid transmits the hydrodynamic blood pressure from the membrane to the sensor.

Current polymer-based pressure sensor encapsulations are subject to drift, offset error, and potential damage to the membrane due to overpressure induced by the sealing of the cavity during the assembly process.
This overpressure originates from the applied stress/strain to the membrane caused by the injection of transmission fluid during the sealing process. The overpressure decays over time caused by the relaxation of the membrane as a result of its viscoelastic/plastic properties.

This may cause an initial pressure offset error, the decay of which causes drift over time. Common sealing techniques do not accommodate this problem as they are designed for significantly larger membranes, less prone to overpressure by liquid injection, or non-viscoelastic/plastic membrane materials, which do not express drift under stress.

Furthermore, current pressure sensor encapsulations are subject to significant temperature cross sensitivity due to the mismatch of thermal expansion coefficients of the transmission fluid and the material of the body forming the cavity. Here, stress/strain is induced on the membrane by unequal volumetric expansion of the transmission fluid and the membrane and cavity materials. Additionally, this is a further source of drift related to the viscoelastic/plastic properties of the membrane.

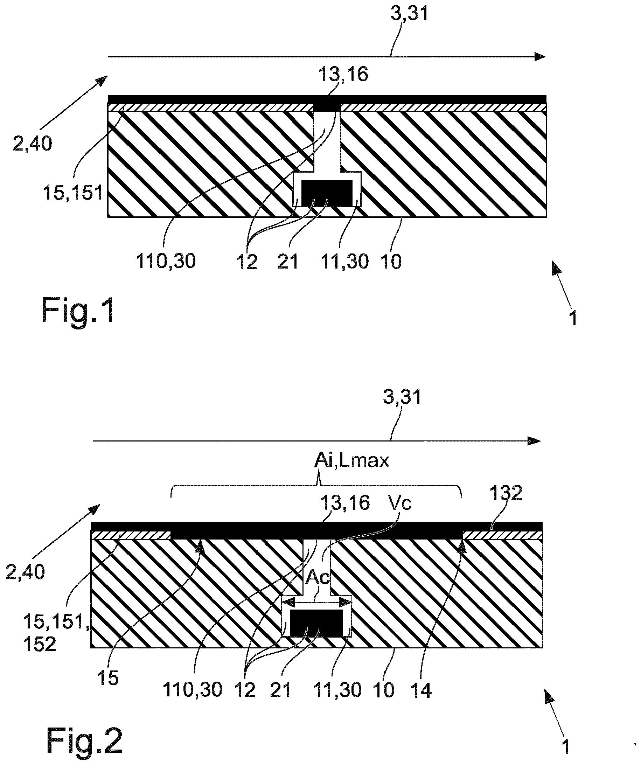

Figure 1 shows a sectional view of a conventional device 1 for measuring the pressure of a fluid 3, in particular blood. Here, the device 1 comprises a support member 10 forming the scaffold structure of the device 1, wherein the support member 10 borders at least on one side the fluid 3, in particular extends circumferentially radial to the direction of flow 31, thereby enclosing an inner space 40 and forming part of a conduit member 2. The surface facing the inner space 40 of the conduit member 2 forms an abutment surface 15, on which a coating 16 in form of a membrane 13 is adhered to by means of nanostructure enhanced forces in a nanostructured zone 151. Here, in this conventional design, abutment surface 15 and nanostructured zone 151 are essentially the same area. Furthermore, a fluidic connection 110 is provided interrupting the abutment surface 15 and extending into the body of the support member 10 fluidically merging into a cavity 11. In the cavity 11 a sensor element 21 is arranged, realising together with the cavity 11, a pressure transmission fluid and the membrane 13 a sensor device 12. The cavity 11 and the fluid connection 110 are filled with a pressure transmission fluid 30. The membrane 13 adhering to the abutment surface 15 by means of nanostructure enhanced forces, sits on an entire nanostructured zone 151, therefore covering the abutment surface 15 as well as the fluid connection 110 interrupting the nanostructured zone 151, realizing a separation of the inner space 40 of the conduit member 2 and the cavity 11.

In use of the conventional device 1, the fluid 3, in particular blood, flows in the direction of flow 31 and through the inner space 40 of the conventional device 1, thereby passing its static pressure to the pressure transmission fluid 30 within the fluid connection 110 and cavity 11 via slight displacements of the membrane 13 perpendicular to the plane formed by the membrane 13, resulting in a pressure change in the pressure transmission fluid 30 which can be measured by the sensor element 21.

In this conventional design the required freedom of movement of the coating 16 is limited due to the extensive and rigid interlocking of the membrane 13 well up to the fluid connection 110 with the nanostructured zone 151 potentially leading to unwanted thermal cross-sensitivity of the components used when the pressure transmission fluid 30 is exposed to temperature differences.

Moreover, space constraints in implants can often prevent the placement of the sensor at the position where the pressure signal is to be measured.

The measurement of pressure inside flow-through inflow cannulas with sensing interfaces parallel to the flow provides only the static component of the pressure. The dynamic component is depending on the cannula's diameter and blood flow rate. A deduction of the total pressure of interest is not possible without knowledge of the flow rate. Therefore, a solution is needed to deduce total pressure from static pressure measurements.

The problems outlined above are partially solved by different embodiments of common devices for measuring pressure of a fluid.

For instance, the encapsulation of the sensor typically relies on either a ball sealing based on compression or flash welding.

The ball sealing is a radial seal based on a forced fit between a ball and a channel having a diameter smaller than the ball. The ball is forced into the channel after the cavity is filled with a pressure transmission liquid creating a seal due the size mismatch between the channel and the ball. During this process a volume of oil corresponding to the channel diameter and ball insertion length is pushed into the cavity creating an overpressure inside of the cavity.

When flash welding the ball to the channel, the ball is lightly pressed against the channel and a short high current pulse is applied from the ball to the surrounding housing. The small contact area between the ball and the channel creates a high electrical resistance and is instantaneously heated up so that the two contacting parts locally melt and join, thus forming a sealing. During this process the pressure transmitting fluid is equally heated up locally, causing a high amount of overpressure.

Furthermore, multiple approaches for integrating a membrane and sensor in a cannula have been described in literature and are herein split into two categories: measurement of the deformation of the inflow cannula body or integration of an encapsulated sensor into the inflow cannula.

Regarding the former category, a part of the wall of the cannula can be thinned down in a small membrane-like area. Due to its very low thickness, the area exhibits detectable deformation under physiological pressures. A strain-gauge is then attached on the outside of the membrane to record the deformation under pressure.

Alternatively, a softer material is chosen to replace the inflow canula and the deformation is converted to changes in wavelength by a Bragg-grating in an optical fibre wrapped around the cannula.

The other category consists of approaches in which a cut-out is realised in the inflow cannula. The cut-out is either filled with the pressure transmission membrane of a previously encapsulated pressure sensor, or with a patterned membrane for Fabry Perot interferometry.

A hybrid approach is a membrane, integrated as part of a complete coating of a cannula covering a cut-out of its wall. The resulting cavity is used to integrate a pressure sensor behind the membrane.

Directly measuring the deformation of the inflow cannula yields the advantage of a well-defined, smooth blood contacting surface. However, such an embodiment of a ventricular assist device is of high cost and time-consuming to manufacture, combined with low signal fidelity and drift.

While the second category offers simpler manufacturing and better signalling, it neglects hemodynamic effects on the non-smooth blood-contacting interface, which may give rise to undesired effects such as blood damage and thrombus formation.

The need to place the sensor directly at the measuring position prevents the placement of measuring positions in thin-walled areas of tubular structures due to lack of space. Recent studies on pump parameter-based flow estimation revealed limitations in accuracy and robustness of such approaches. This also limits the applicability of such estimated flows for dynamic pressure estimation and the estimation of the total pressure based on static, intra cannula pressure recordings.

A further ventricular assist device (VAD) cannula is known from the US patent US 9,744,282 B2 including multiple independent sensors which may help decrease the incidence of ventricular collapse and provide automatic speed control. The cannula may include two or more independent sensors. One sensor may measure ventricular pressure, while another may measure ventricular volume and/or ventricular wall location.

The objective of the invention is to provide a device for measuring pressure of a fluid and a tubular structure as well as a method for manufacturing said device and a method for measuring pressure of a fluid, with which the measurement of pressure is possible in a simple and cost-effective manner, even under changing environmental parameters.

This objective is solved by the device for measuring the pressure of a fluid according to claim 1, by the tubular structure of claim 12 and by the method for manufacturing said device according to claim 14, as well as by the method for measuring the pressure of a fluid according to claim 18.

Advantageous embodiments of the device according to the invention are specified in subclaims 2-11. Advantageous embodiments of the method for manufacturing a device for measuring pressure of a fluid according to the invention are specified in subclaims 15-15.

### DESCRIPTION

In one aspect the invention provides a device for measuring pressure of a fluid, in particular of liquid like blood, comprising at least one sensor device in a support member comprising a cavity filled or fillable with a pressure transmission fluid, in particular a working liquid, and inside the cavity a sensor element, wherein the cavity is closed on one side by a membrane. The membrane abuts against an abutment surface of the support member and at least one rim of the membrane is fixed to the abutment surface. An inner area restricted on its circumference by the zone of fixation of the membrane to the abutment surface is not fixed to the abutment surface, so that the inner area of the membrane is movable with regard to the abutment surface and can elevate off from the abutment surface.

The membrane is slightly flexible and therefore moveable in a way essentially perpendicular to the plane established by the membrane, therefore, as the blood pressure changes, the movable membrane can move slightly towards or away from the sensor, so that the pressure transmission fluid inside the cavity is subject to a change of pressure, which can be detected by the sensor device in the pressure transmission fluid.

That is, during elevation the distance filled with pressure transmission fluid between the membrane and the abutment surface is increased.

The device according to the invention enables a low temperature cross sensitivity and consequently low drift of measured pressure values, while exploiting a high interface quality and simplicity.
It is possible to manufacture the device according to the invention at a first temperature and to use it in a second temperature range, which differs from the first temperature, without temperature expansion-related volume fluctuations of the pressure transmission fluid being able to lead to different pressure values of the pressure transmission fluid, which can influence the pressure measurement of the fluid.

In an advantageous embodiment, said membrane is fixed to the abutment surface by means of nanostructure force in a nanostructured zone of the abutment surface, realised by a form-fit connection between a plurality of protrusions on the support member penetrating into the material of the membrane. In particular, the membrane is fixed to the abutment surface solely by nanostructure force.

Due to the fact that the membrane is attached to the abutment surface by means of nanostructure force, a smooth transition of the coating results, so that no disturbances of blood flow are generated.

Furthermore, the nanostructure-based interlocking achieves a high durability.

The protrusions may be formed as a plurality of pins, having a length between 1*10⁻⁹ to 1*10⁻⁷ m. Additionally to the form-fit connection a substance-to-substance bond may be provided as well between the protrusions and the material of the membrane, creating a mechanical interlocking by means of adhesion.

In case of Parylene as the material of the membrane, the protrusions may be enclosed by Parylene by means of evaporation and deposition of Parylene on the protrusions.

By locally controlling the growth/remove grown nanostructures and/or by tuning the interlocking between the coating material and the nanostructure, for instance by changing the nanostructure shape or by blocking the empty space in the nanostructures, areas of varying interlocking between the abutment surface and the coating material can be achieved. Thereby, the nanostructure may also be arranged only partially or not area-wide in the nanostructured zone. In particular, the nanostructure can be arranged in said nanostructured zone with one or more patterns on individual surface areas.

In one embodiment, the membrane is a part of a coating covering the abutment surface.

That is, the membrane is realised in the same plane as the overall coating of the support member, such that there are no seams.

The membrane may be aligned with its longitudinal side along the direction of flow of the blood in a tubular structure, for instance of a Ventricular Assist Device.

Along this direction, the membrane has a maximum extension of 10mm-16mm, particularly 12mm-14mm, and a maximum extension in the transverse direction of 2-5mm, particularly 3mm-4mm.

The thickness of the membrane can be, for example, 20 µm.

The Young's modulus of the membrane is, for example, 2,76* 10¹² N/mm².

Furthermore, in an advantageous embodiment the device for measuring pressure of a fluid comprises a support member which is part of a conduit member adapted to lead a fluid inside the device.

That means that the support member can be a part of a tube leading blood inside of a tubular structure, like a cannula of a Ventricular Assist Device.

Moreover, the ratio of the inner area Ai to the area of a cross section Ac of the cavity may be at least Ai/Ac = 10 to 30, in particular Ai/Ac = 15 to 25.

The cross-section Ac of the cavity is to be measured parallel to the plane of the membrane.

This ensures that in the event of temperature-related expansion of the pressure transmission fluid, the membrane in the non-fixed area can be lifted minimally from the abutment surface, so that the volume of the cavity can increase significantly without causing a significant pressure increase in the pressure transmission fluid, which could cause drift of the measured pressure due to creep of the polymer membrane. Thus, no drift phenomena of the measured pressure values can occur.

The volume of the cavity, e.g., with the membrane in contact with the abutment surface, may be at most 1,7x10⁻⁸ m³, for instance 1.65304x10⁻⁸ m³.

The inner area Ai may be 4 to 6 x 10⁻⁵ m², for instance 4.462 x 10⁻⁵ m².

The ratio between the volume Vc of the cavity and the inner area Ai may be Vc / Ai = 3 to 4 x 10⁻⁴ m; in particular 3,7 x 10⁻⁴ m.

Further, the ratio between the volume Vc of the cavity and the longest extension Lmax of the inner area Ai may be Vc / Lmax = 15 to 20 mm².In a further advantageous embodiment, the device for measuring pressure a of a fluid comprises a pressure transmission fluid inside the cavity, in particular a liquid having a first volumetric thermal expansion coefficient α1, and the material of the membrane has a second volumetric thermal expansion coefficient α2, wherein the ratio of the thermal expansion coefficients is α1/α2 = 1 to 12.

In particular, the ratio of the thermal expansion coefficients may be α1/α2 = 8 to 12. For instance, the first volumetric thermal expansion coefficient α1 of the working liquid may be 1.04E-03, and the second volumetric thermal expansion coefficient α2 of the membrane may be 1.05E-04.

The pressure transmission fluid is therefore a fluid for transmitting a pressure to the sensor device. The pressure of the pressure transmission fluid depends on the pressure of the fluid to be measured, for instance on the blood pressure. That is, the pressure of the pressure transmission fluid depends on the volume provided for the pressure transmission fluid which in turn is dependent on the minimal possible deformation of the membrane with regard to the abutment surface, which may slightly occur in dependency of the pressure of the pressure of the fluid to be measured.

The material of the membrane is for instance a polymer, in particular a Parylene, and the material of the support member is for instance a Titanium.

In particular, the material of the membrane may be a Parylene C, and the material of the support member may be a Titanium grade 5.

Membranes made of Parylene C have the advantage of relatively low bending resistance, so that said membranes are easily deformable. The slight change in volume of the cavity caused by deformation of the membrane leads to changes of pressure in the pressure transmission fluid detectable by the sensor.

The invention provides a so-called free-shape membrane embedded in the coating material of the conduit member.

This free shape membrane solution allows efficient membrane fabrication and combines high signal fidelity, low drift and optimal hemodynamic properties of the sensing interface.

The inner area of the membrane may have a shape in the form of an ellipse or a Lame oval.

An outer boundary of the membrane can also have this oval shape, so that the diaphragm can optimally adapt to a concavely curved inner side of a tube.

Alternatively, the inner area of the membrane may have a a surface consisting of a rectangle combined with semicircles on opposite sides of the rectangle.

In an advantageous embodiment, the device for measuring pressure of a fluid comprises a recess within the support member, said recess being fluidically connected to the cavity via an opening, wherein in the recess a flexible element is arranged, which rests with at least one wall portion against an inner wall of the recess, wherein in the flexible element a pressure element is arranged, which presses at least one wall portion of the flexible element against the inner wall of the recess, so that the opening is sealed liquid-tightly.

Furthermore, the flexible element may be a hollow cylinder closed on one side, and the pressure element may be a ball.

The hollow cylinder closed on one side is a so-called cup.

The bending strength of the wall portion is significantly lower than the compression strength of the pressure element, so that the pressure element can press the wall portion against the inner wall, thus realising a fluid seal between the flexible element and the material of the support member forming the recess.

Advantageous in this design is that after filling the cavity with pressure transmission fluid, and during closing the recess by inserting the flexible element, redundant pressure transmission fluid can be displaced out of the recess and can thereby escape from the recess laterally past the flexible element.

The flexible element thereby does not seal the recess or its opening, since it is moved away from the inner wall by the pressure transmission fluid due to its relatively low bending stiffness in the region of its contact with the inner wall of the recess, so that the pressure transmission fluid can escape between the inner wall and the wall portion. This ensures that the pressure transmission fluid is not under an increased pressure in relation to the ambient pressure, which would lead to an undesired influence on the pressure measurement values when using the VAD.

A seal of the recess or its opening is achieved by inserting the pressure element into the flexible element so that the wall portion of the flexible element is pressed firmly against the inner wall of the recess and due to the elastic property of the pressure element, the flexible element and/or the support member permanently maintains this pressure.

For flow measurement, multiple sensors in flow direction are possible.

The design of the device for measuring pressure of a fluid having the recess and the flexible element in the recess may comprise at least one sensor device in a support member comprising a cavity filled or fillable with a pressure transmission fluid, in particular a working liquid, and inside the cavity a sensor element. However, this device for measuring pressure of a fluid may not comprise the further features of claim 1. Hence, the device for measuring pressure of a fluid having the recess and the flexible element in the recess may be carried independent from the invention as claimed by claim 1.

This embodiment of the present invention allows to seal a fluid volume, in particular pressure transmission fluid filled volume, without significant overpressure.

The cup-sphere seal ensures fluid tightness together with low production costs at perpetuation of the same pressure in the pressure transmission fluid.

For this, a flexible element as a hollow cylinder closed on one side, essentially having the shape of a cup, is tightly fit to the recess through which the volume is filled with fluid, is slowly inserted into the recess allowing excess fluid to creep out of the recess and any overpressure to decay. Spreading the wall of the hollow cylinder towards the walls of the recess by pressing a pressure element in form of a ball into the hollow cylinder drastically reduces the amount of fluid pressed into the cavity during the sealing process and therefore also minimizes pressure increase inside the cavity.

With this, damage of the seal and also of the membrane is avoided, and drift of measured pressure values is reduced.

Hence, this embodiment provides a miniaturized hermetic seal, while minimizing the injected fluid volume during the sealing process.

The proposed embodiment of the device according to the invention enables the cost-efficient sealing of fluid filled cavities at room temperature, without specialized equipment, and ensures that only the exact volume of fluid is contained inside of the cavity.

Additionally, or alternatively to the claimed device for measuring pressure of a fluid and the embodiments mentioned above, a device for measuring pressure of a fluid can have a fluid connection on an inner side of the conduit member, which forms one end of a fluid-filled transmission channel, which in turn is fluidically connected to a cavity, in which a sensor unit is arranged. In this case, the distance between the fluid connection and the cavity or the sensor unit is very large.

Here, once again, it is provided that the fluid connection or the transmission channel is sealed fluid-tight from the inner space of the conduit member by means of a membrane, in particular with a membrane arranged according to the invention. In particular, it is provided that this distance is at least as large as half the diameter or half the maximum radial extension of the inner space formed by the conduit members through which fluid is to flow, measured transversely to the direction of flow.

This has the advantage that the sensor unit can be positioned nearly independent of location in relation to the fluid connection, so that the position of the fluid connection is very flexible.

If necessary, a network of transmission channels can connect a fluid connection with several cavities and sensor units for multiple measurements, or a cavity with a sensor unit arranged therein can be fluidically connected via multiple transmission channels with multiple fluid connections for averaging the determined measured values.

In particular, a respective cavity can be integrated in a housing forming a conduit member.

In particular, this housing can be produced by means of an additive manufacturing, in particular by a 3D printing process, including the at least one transmission channel.

That is, the device according to the inventive solution may comprise a free-form network of transmission channels, implemented in the support member, for instance manufactured by 3D printing. These embedded transmission channels can transmit pressure from remote areas to one or more sensors, even when sensor placement is constrained by space.

Hence, said transmission channel network can be used to measure the signal from a specific location with multiple sensors or to measure averaged signals of multiple points with a single sensor or multiple sensors.

In this embodiment of a device for measuring pressure of a fluid, a combined measurement of blood pressure and blood flow in a tubular structure, for instance in the inflow cannula of a ventricular assist device may be carried out, in particular by two or more pressure sensors placed along a tapered profile of the inflow cannula of the conduit member, wherein the sensors may be located at different radii of the tapered cannula. The total pressure and flow rate can be calculated from the static pressure values measured by the two or more pressure sensors.

In this embodiment of a device for measuring pressure of a fluid, a nearly unlimited access to the cannula's abutment surface for pressure sensing is provided. This allows easy implantability of the Ventricular Assist Device due to its compact shape, which is made possible by the free-form transmission channel network mentioned above.

The device for measuring pressure of a fluid according to the invention eliminates flow rate dependent pressure signal errors and enables accurate, integrated flow measurement in the inflow cannula, without the need of additional, dedicated measurement systems and additionally enables pressure measurement at almost arbitrary positions in the device independent of the positioning of the sensor element, even at positions where the thickness of the support member is not sufficient to accommodate a sensor element.

In a further aspect, the invention relates to a tubular structure allowing a flow-through of blood, in particular to a mechanical circulatory support like a ventricular assist device or an inflow device, comprising at least one device for measuring pressure of a fluid according to the invention.

In one embodiment the tubular comprises at least two devices for measuring pressure of a fluid positioned along the flow path of the fluid to be measured at two sensing locations having different cross-sections of the tubular structure. Here, the total pressure and/or flow rate of the fluid through the device may be calculated based on static pressure measurements at these two sensing locations. At constant total pressure and flow rate the static pressure measured at said locations with different cross-sections of the flow path, differ from each other as the difference in cross-sections causes a difference in flow-velocity (mass conservation) which in turn introduces an increase in dynamic pressure at the sensing location with the smaller cross-section of the flow path. As the measured static pressure is determined by difference of the constant total pressure and the flow-dependent dynamic pressure, it will change with a change in flow rate. This effect is stronger for sensing sites with a smaller cross-section of flow. Therefore, the difference in static pressure at two measurement points with different and known flow cross-sections clearly defines the instantaneous total pressure and flow rate.

In another aspect, the invention relates to a method for manufacturing a device for measuring pressure of a fluid according to the invention, the method comprising the steps of: providing a support member comprising a cavity fillable with a pressure transmission fluid; providing a membrane; positioning the membrane on the abutment surface of the support member in such a manner that the membrane abuts against the abutment surface; connecting of at least a rim of the membrane to the abutment surface, so that the cavity is closed on one side by the membrane and an inner area restricted on its circumference by the zone of fixation of the membrane to the abutment surface is not fixed to the abutment surface, so that the inner area of the membrane is movable with respect to the abutment surface.

Additionally, at the same time as at least one of the above steps or later, a sensor element can be placed in the cavity and/or pressure transmission fluid can be filled into the cavity.

In this method a part of the coating of the conduit member is transformed into a membrane, fully integrated in the coating layer, wherein the coating easily delaminates and realizes a seamless and controllable part of the coating material usable as a membrane.

According to this method the temperature cross sensitivity can be reduced and by that also additional drift phenomena related to stress and viscoelastic relaxation.

In an advantageous embodiment of the method for manufacturing a device for measuring pressure of a fluid nanostructure is generated on an abutment surface of the support member, and at least a rim of the membrane is connected to the abutment surface by means of nanostructure force in a nanostructured zone of the abutment surface, wherein the strength of the nanostructure force is adjusted by
i) controlling the growth of the nanostructure;
ii) removing grown nanostructure;
iii) changing the nanostructure shape, and/or
iv) blocking empty spaces of the nanostructure.

Furthermore, according to an advantageous embodiment of the method for manufacturing a device for measuring pressure of a fluid, the nanostructures of the nanostructured zone are generated on the abutment surface by means of the following steps: immersing at least the abutment surface of the support member at least partially in an alkali solution at a first temperature T1, cooling the support member to a second temperature T2, wherein T1 ≥ T2+ 160 K.

Additionally, and/or optionally, the nanostructured zone to be generated of the substrate member may be polished, such that the nanostructured zone to be generated is sufficiently flat.

The first temperature T1 is held a sufficient amount of time, preferably of at least 6h.

The second temperature T2 may be room-temperature.

Further, before alkali treatment of the nanostructure zone to be generated, the abutment surface is at least partially polished.

In an embodiment, thereafter washing and calcinating the support member comprises the nanostructures of the nanostructured zone.

In an embodiment of the method for manufacturing a device for measuring pressure of a fluid, a modified inflow cannula of the support member has a conical inner shape and a biocompatible coating, mechanically interlocked with the abutment surface of the support member, and sensors placed in cavities in the support member. Areas of no interlocking between the coating and the abutment surface of the support member allowing the coating to locally delaminate and form a diaphragm. These areas are connected to the cavities by a small fluid-filled channel. The blood pressure transmits via the diaphragm and channel to the sensor cavity where it can be measured.

In an embodiment of the device for measuring pressure of a fluid, an additional channel connects the fluid-filled cavity and is used to fill the cavity with fluid, in particular with pressure transmission fluid. The additional channel ends in a recess formed in the support member.

To seal-in the fluid, a flexible element in shape of a cup is placed in the recess, displacing the fluid in the recess. A fluid-tight sealing is achieved by pressing the cup's walls against the recess's walls by inserting a pressure element, e.g., a ball, into the cup.

An advantageous embodiment of the method for manufacturing a device for measuring pressure of a fluid refers to a recess in the support member, said recess being fluidically connected to the cavity via an opening, wherein a flexible element is arranged in the recess so that it abuts with at least one wall portion against the inner wall of the recess, so that surplus pressure transmission fluid may leak out of the recess during assembly. Thereafter, a pressure element is arranged in the flexible element, which presses at least one wall portion of the flexible element against the inner wall of the recess, so that the opening is sealed in a fluid-tight manner.

A further aspect of the present invention relates to a method for measuring pressure of a fluid, in particular of blood, wherein a device for measuring pressure of a fluid, in particular liquid like blood, is provided, said fluid being led along the membrane such that the membrane moves slightly in dependency of the pressure of the fluid and the volume of the cavity is slightly changed, and the pressure of the pressure transmission fluid inside the cavity is measured.

In one embodiment, the total pressure and/or flow rate of the fluid through the device is calculated based on static pressure measurements at at least two sensing locations that are positioned at locations with different cross-sections along the flow path of the fluid to be measured.

At constant total pressure and flow rate the static pressure measured at said locations with different cross-sections of the flow path, differ from each other as the difference in cross-sections causes a difference in flow-velocity (mass conservation) which in turn introduces an increase in dynamic pressure at the sensing location with the smaller cross-section of the flow path. As the measured static pressure is determined by difference of the constant total pressure and the flow-dependent dynamic pressure, it will change with a change in flow rate. This effect is stronger for sensing sites with a smaller cross-section of flow. Therefore, the difference in static pressure at two measurement points with different and known flow cross-sections clearly defines the instantaneous total pressure and flow rate.

The method for measuring pressure of a fluid may be carried out inside or outside of the human or animal body. In a special embodiment of this method, the device for measuring pressure of a fluid can be in fluidic connection with the human or animal body, or without fluidic connection with the human or animal body.

The invention is further described below with reference to the embodiments shown in the appending drawings.

### DESCRIPTION OF FIGURES

Fig. 1 shows a sectional view of a conventional device for measuring the pressure of liquids, in particular blood.
Fig. 2 shows a sectional view of an embodiment according to the invention in a first state.
Fig. 3 shows a sectional view of an embodiment according to the invention in a second state.
Fig. 4 shows a sectional view of another embodiment according to the invention in a first state.
Fig. 5 shows the embodiment of figure 4 in a second state.
Fig. 6 shows the embodiment of figure 4 in a third state.
Fig. 7 shows a sectional view of a further embodiment of the device according to the invention.

Figure 1 has already been referred to as description of the state of the art.

Figure 2 shows a sectional view of the device 1 according to the invention in an undelaminated state. The device 1 comprising a support member 10 borders at least on one side the fluid 3, in particular extends radial to the direction of flow 31 of the fluid 3, thus forming part of a conduit member 2, and thereby enclosing an inner space 40, wherein the support member 10 has a fluid connection 110 recessed therein which interrupts an abutment surface 15 and fluidically merges into a cavity 11. In said cavity 11, a sensor element 21 is provided, realising together with the cavity 11 and the membrane 13 a sensor device 12. Said fluid connection 110 and cavity 11 are filled with a pressure transmission fluid 30. At least a part of the surface of the support member 10 facing the inner space 40 of the conduit member 2 forms the abutment surface 15, to which a coating 16, e.g., a membrane 13, adheres solely by nanostructure forces in a nanostructured zone 151 which forms a zone of fixation 152. The membrane 13 otherwise abuts the abutment surface 15 in an undelaminated state as shown in figure 2, thereby also covering an area of the abutment surface 15 restricted by a circumference 14 and therefore also covering the opening of the fluid connection 110 recessed in the support member 10. In this exemplary illustration of an embodiment, abutment surface 15 on the one hand and nanostructured zone 151 or zone of fixation 152 on the other hand cannot be used interchangeably and nanostructured zone 151 and zone of fixation can be used interchangeably. Here, an inner area Ai restricted on its circumference 14 and by the zone of fixation 152 is not fixed to the abutment surface 15. The inner area Ai of the membrane 13 with which the membrane 13 abuts the abutment surface 15 in an undelaminated state has a rim 132 extending in a distance around the opening of the fluid connection 110 along the circumference 14, the rim 132 spanning the area of the abutment surface 15 without nanostructures and therefore without nanostructure force. It should be noted that the circumference 14 represents the transition zone at which the rim 132 of the membrane 13 adheres to the nanostructured zone 151 of the abutment surface 15 by means of nanostructure forces and the membrane 13 abuts in the shown undelaminated state to the abutment surface 15. Furthermore, the inner area Ai restricted by the circumference 14 is larger than the area Ac of the cross section of the cavity 11.

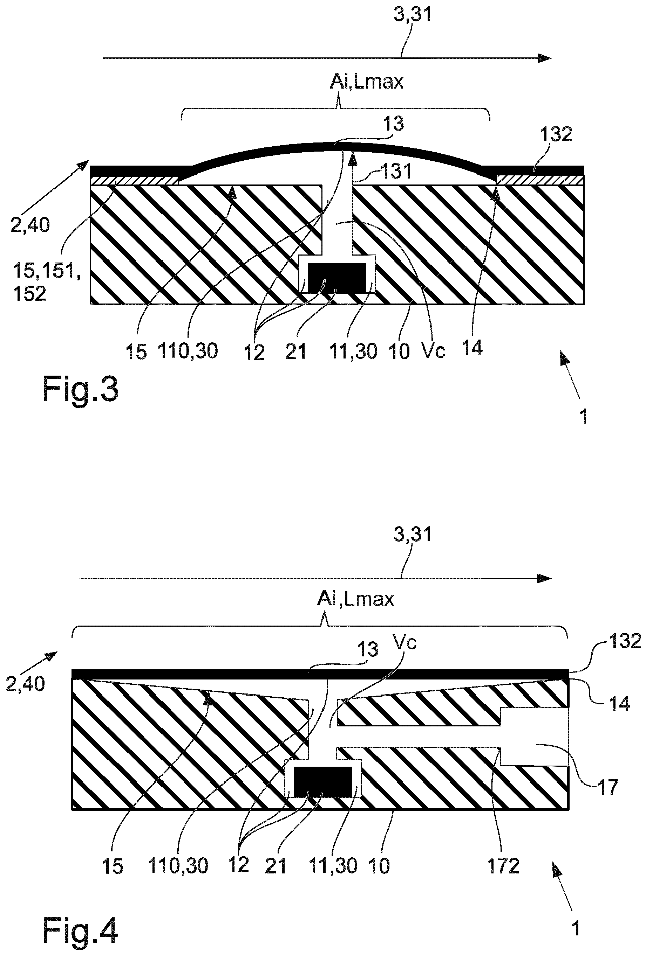

Figure 3 shows a sectional view of the device 1 of figure 2 according to the invention in a delaminated state. Here, the inner area Ai of the membrane 13, which in figure 2 abuts the abutment surface 15, is arched into the inner space 40 of the conduit member therefore not abutting the abutment surface 15 in a delaminated state, due to a pressure difference between the fluid 3 and the pressure transmission fluid 30. It should be noted that membrane 13 is essentially seamless when transitioning from the nanostructured zone 151 of the abutment surface 15 across the circumference 14 to the area of the abutment surface 15 without nanostructures to enable a delaminated state as shown in figure 2, due to the freedom of movement and wider inner area Ai of the membrane 13 and compared to the conventional state shown in figure 1.

In use of the device 1, the fluid 3 flows in the direction of flow 31 through the inner space 40 of the conduit member 2 of the device 1, thereby passing its pressure to the pressure transmission fluid 30 within the fluid connection 110 fluidically connected with the cavity 11 via movement of the membrane 131 perpendicular to the plane formed by the membrane 13 in a resting position, resulting in a pressure difference of the pressure transmission fluid 30 which can be measured by the sensor element 21.

Due to the larger volume provided on the side of the membrane 13 facing the cavity 11, the membrane 13 can easily elevate from the abutment surface 15 as the temperature increases and therefore provide additional volume for the pressure transmission fluid 30, such that the pressure of pressure transmission fluid 30 is only slightly or essentially not changed.

Figure 4 shows a sectional view of another embodiment according to the invention. The device 1 comprising a support member 10 borders at least on one side the fluid 3, in particular extends radial to the direction of flow 31 of the fluid 3, thereby enclosing an inner space 40 of the conduit member 2, wherein the support member 10 has a fluid connection 110 recessed therein which interrupts the slightly inwards falling abutment surface 15 without nanostructures and merges into a cavity 11. Thereby, a conical space is provided below the membrane 13, thus providing a large volume fillable with the pressure transmission fluid 30. In said cavity 11 a sensor element 21 is provided, realising together with the cavity 11 and the membrane 13 a sensor device 12. Said fluid connection 110 and cavity 11 are filled with a pressure transmission fluid 30. Additionally, a recess 17 is provided within the body of the support member 10 and extending parallel to the direction of flow, wherein the recess 17 is fluidically connected to the cavity 11 and wherein the fluid connection 110, cavity 11 and recess 17 are fluidically connected and fillable by means of injection into an opening 172 of the recess 17 with the pressure transmission fluid 30. Furthermore, as described in figures 2 and 3 the inner area Ai of the membrane 13 restricted by its rim 132 is shown.

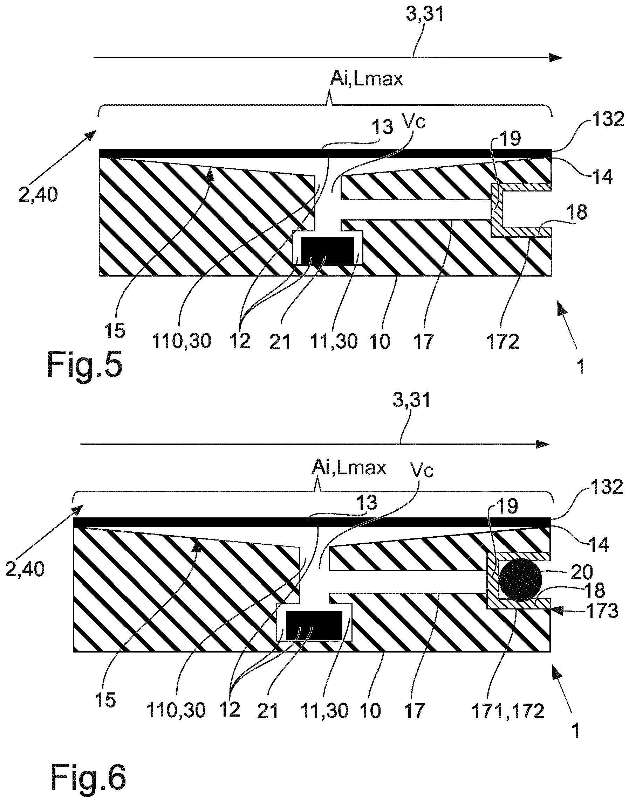

Figure 5 shows a sectional view of the embodiment according to the invention as shown in figure 4, wherein in addition to the embodiment shown in figure 4, a flexible element 18, here shaped as a cup, is provided which is introduced into the recess 17 essentially form-fittingly closing the opening 172 of the recess 17, however, in such a way that the injected pressure transmission fluid 30 is still able to escape out of the 172 due to the flexibility of the wall portions 19 of the flexible element 18, thereby decreasing a possible overpressure of the pressure transmission fluid 30 acting on the membrane 13.

Figure 6 shows the sectional view of an embodiment according to the invention as shown in figures 4 and 5, wherein additionally to the embodiment shown in figure 5, a pressure element 20, e.g. a ball is provided, wherein the pressure element 20 is placed in the hollow space of the flexible element 18 and is pressed against the wall portions 19 of the flexible element 18, such that the wall portions 19 of the flexible element 18 are pressed against one or more inner walls 171 of thereby reducing an interspace 173 between the flexible element 18 and the recess 17 such that a fluid-tight barrier is formed.

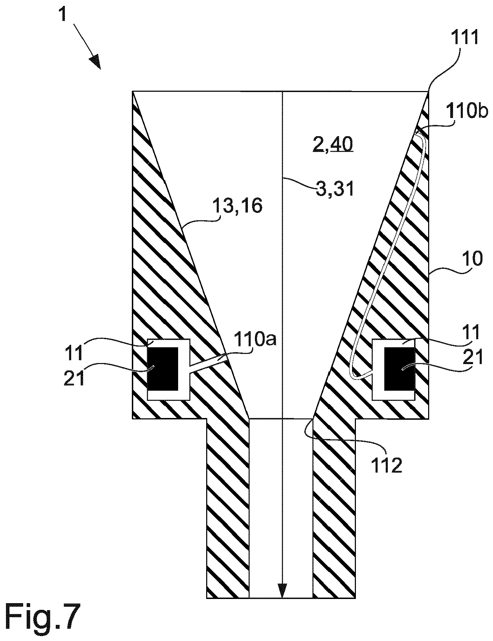

Figure 7 shows a sectional view of another embodiment of the device 1 according to the invention, wherein the scaffold structure of the device 1 is formed by the support member 10 extending radial to the direction of flow 31 of a fluid 3, enclosing an inner space 40 of the conduit member 2. The conduit member 2 and therefore the support member 10 extend radially at one end 111 with a greater inner diameter than at a second end 112 forming the inner space 40 as a conical shape. Furthermore, the support member 10 has at least two cavities 11 with a respective sensor element 21 arranged therein, wherein each cavity 11 is fluidically connected with a respective fluid connection 110, and wherein a respective cavity 11 and fluid connection 110 are filled with a pressure transmission fluid 30. The fluid connection 110 is formed such that it interrupts the abutment surface 15 and merges into the cavity 11 via an essentially shortest path or that it interrupts the abutment surface 15 at an essentially arbitrary distance with respect to the cavity 11, e.g. at the one end 111 with a greater diameter or in other words, in areas at which the thickness of the support member 10 is not sufficient in order to house a cavity 11 with a sensor element 21 arranged therein, so that measurements of pressure can be performed by the sensor element 21 even though the sensor element 21 not being in close proximity to the area of transmission of fluid pressure. In this embodiment the coating 16 as well as the membrane 13 may be provided as illustrated and described in figures 2-6. However, the present invention is not restricted to a combination of coating 16 as well as the membrane 13 as illustrated and described in figures 2-6 together with the embodiment of figure 7.

Due to the conical shape, the static pressure of the streaming fluid to be measured is smaller at the first fluidic connection 110a at the one end 111 than at the second fluidic connection 110b at the second end 112. Based on the difference of measured static pressure values the total pressure and/ or the flow rate of the fluid 3 may be calculated.

### Reference listing

- 1: device
- 2: conduit member
- 3: fluid
- 10: support member
- 11: cavity
- 12: sensor device
- 13: membrane
- 14: circumference
- 15: abutment surface
- 16: coating
- 17: recess
- 18: flexible element
- 19: wall portion
- 20: pressure element
- 21: sensor element
- 30: pressure transmission fluid
- 31: direction of flow
- 40: inner space
- 110a: first fluidic connection
- 110b: second fluidic connection
- 111: one end
- 112: second end
- 131: movement of the membrane
- 132: rim
- 151: nanostructured zone
- 152: zone of fixation
- 171: inner wall
- 172: opening
- 173: interspace
- Ai: inner area
- Ac: area of the cross section of the cavity

## Claims

1. Device (1) for measuring pressure of a fluid (3), in particular of liquid like blood, comprising at least one sensor device (12) in a support member (10) comprising a cavity (11) filled or fillable with a pressure transmission fluid (30), in particular a working liquid, and inside the cavity (11) a sensor element (21), wherein the cavity (11) is closed on one side by a membrane (13), wherein the membrane (13) abuts against an abutment surface (15) of the support member (10) and at least one rim (132) of the membrane (13) is fixed to the abutment surface (15), and an inner area (Ai) restricted on its circumference (14) by the zone of fixation (152) of the membrane (13) to the abutment surface (15) is not fixed to the abutment surface (15), so that the inner area (Ai) of the membrane (13) is movable with regard to the abutment surface (15) and can elevate from the abutment surface (15).

2. Device (1) for measuring pressure of a fluid (3) according to claim 1, **characterized in that** the membrane (13) is fixed to the abutment surface (15) by means of nanostructure force in a nanostructured zone (151) of the abutment surface (15), in particular solely by nanostructure force, realised by a form-fit connection between a plurality of protrusions on the support member (10) penetrating into the material of the membrane (13).

3. Device for measuring pressure of a fluid (3) according to one of the preceding claims, **characterized in that** the membrane (13) is part of a coating covering the abutment surface (15).

4. Device (1) for measuring pressure of a fluid (3) according to one of the preceding claims, **characterized in that** the support member (10) is part of a conduit member (2) adapted to lead a fluid (3) inside the device (1).

5. Device (1) for measuring pressure of a fluid (3) according to one of the preceding claims, **characterized in that** the ratio of the inner area Ai to the area of a cross section Ac of the cavity (11) is at least: Ai/Ac = 10 to 30; in particular Ai/Ac = 15 to 25.

6. Device (1) for measuring pressure of a fluid (3) according to one of the preceding claims, **characterized in that** the ratio between the volume Vc of the cavity (11) and the longest extension Lmax of the inner Area Ai is Vc / Lmax = 15 to 20 mm².

7. Device (1) for measuring pressure of a fluid (3) according to one of the preceding claims, **characterized in that** the pressure transmission fluid (30) inside the cavity (11) is a working liquid having a first volumetric thermal expansion coefficient α1, and the material of the membrane (13) has a second volumetric thermal expansion coefficient α2, wherein the ratio of the volumetric thermal expansion coefficients is α1/α2 = = 1 to 12, in particular 8 to 12.

8. Device (1) for measuring pressure of a fluid (3) according to one of the preceding claims, **characterized in that** the material of the membrane (13) is a polymer, in particular a Parylene, and the material of the support member (10) is a Titanium.

9. Device (1) for measuring pressure of a fluid (3) according to one of the claims 4 to 8, **characterized in that** the inner area of the membrane has a shape in the form of an ellipse or a Lame oval.

10. Device (1) for measuring pressure of a fluid (3) according to one of the preceding claims, **characterized in that** in the support member (10) a recess (17) is provided, which is fluidically connected to the cavity (11) via an opening, wherein in the recess (17) a flexible element (18) is arranged, which rests with at least one wall portion (19) against an inner wall (171) of the recess (17), wherein in the flexible element (18) a pressure element (20) is arranged, which presses at least one wall portion (19) of the flexible element (18) against the inner wall (171) of the recess (17), so that the opening (172) is sealed liquid-tightly.

11. Device (1) for measuring pressure of a fluid (3) according to claim 10, **characterized in that** the flexible element (18) is a hollow cylinder closed on one side, and the pressure element (20) is a ball.

12. Tubular structure allowing a flow-through of blood, in particular mechanical circulatory support like a ventricular assist device, comprising at least one device (1) for measuring pressure of a fluid (3) as claimed in one of the claims 1-11.

13. Tubular structure according to claim 12, comprising at least two devices (1) for measuring pressure of a fluid (3) positioned along the flow path of the fluid (3) to be measured at two sensing locations having different cross-sections of the tubular structure.

14. Method for manufacturing a device (1) for measuring pressure of a fluid as claimed in at least one of the claims 1 to 13, comprising the steps of:
- providing a support member (10) comprising a cavity (11) fillable with a pressure transmission fluid (30);
- providing a membrane (13);
- positioning the membrane (13) on the abutment surface (15) of the support member (10) in such a manner that the membrane (13) abuts against the abutment surface (15);
- connecting of at least a rim (132) of the membrane (13) to the abutment surface (15), so that the cavity (11) is closed on one side by the membrane (13) and an inner area (Ai) restricted on its circumference (14) by the zone of fixation (152) of the membrane (13) to the abutment surface (15) is not fixed to the abutment surface (15), so that the inner area (Ai) of the membrane (13) is movable with respect to the abutment surface (15).

15. Method for manufacturing a device (1) for measuring pressure of a fluid (3) according to claim 14, **characterized in**
generating of nanostructure on an abutment surface (15) of the support member (10), and connecting of at least a rim (132) of the membrane (13) to the abutment surface (15) by means of nanostructure force in a nanostructured zone (151) of the abutment surface (15), wherein the strength of the nanostructure force is adjusted by
i) controlling the growth of the nanostructure;
ii) removing grown nanostructure;
iii) changing the nanostructure shape, and/or
iv) blocking empty spaces of the nanostructure.

16. Method for manufacturing a device (1) for measuring pressure of a fluid according to claim 14 and 15, **characterized in that** the nanostructures of the nanostructured zone (151) are generated on the abutment surface (15) by means of the following steps:
- immersing the at least the abutment surface of the support member at least partially in an alkali solution at a first temperature T1,
- cooling the support member to a second temperature T2, wherein T1 ≥ T2+ 160 K.

17. Method for manufacturing a device (1) for measuring pressure of a fluid (3) according to at least one of the claims 14 to 16, **characterized in that** in the support member (10) a recess (17) is provided, which is fluidically connected to the cavity (11) via an opening (172), wherein a flexible element (18) is arranged in the recess (11) so that it abuts with at least one wall portion (19) against the inner wall (171) of the recess (11), so that surplus pressure transmission fluid (30) may leak out of the recess 811) , and thereafter a pressure element (20) is arranged in the flexible element (18), which presses at least one wall portion (19) of the flexible element (18) against the inner wall (171) of the recess (11), so that the opening (172) is sealed in a fluid-tight manner.

18. Method for measuring pressure of a fluid (3), in particular of blood, wherein at least one device (1) for measuring pressure of a fluid (3) according to at least one of the claims 1 to 11 is provided, fluid (3), in particular liquid-like blood, is led along the membrane (13) such that the membrane (13) moves slightly in dependency of the pressure of the fluid (3) and the volume of the cavity (11) is changed, and the pressure of the pressure transmission fluid (30) inside the cavity is measured. 19. Method for measuring pressure of a fluid (3) according to claim 18, wherein the total pressure and/or flow rate of the fluid (3) through the device (1) is calculated based on static pressure measurements at at least two sensing locations that are positioned at locations with different cross-sections along the flow path of the fluid to be measured.
